# EUROPEAN PATENT APPLICATION

(11) **EP 2 601 987 A1**
(43) Date of publication of application: **12.06.2013**
(21) Application number: 11814493.0
(22) Date of filing: 26.07.2011
(51) Int. Cl.: A61M 5/168, A61M 39/00, F16K 15/08

(54) **THREE-WAY VALVE UNIT, NON-RETURN VALVE, AND FLUID MATERIAL TRANSFER DEVICE**

(30) Priority: 06.08.2010 WO PCT/JP2010/063347
(71) Applicant: En Otsuka Pharmaceutical Co., Ltd., Hanamaki-shi, Iwate 025-0312 (JP)
(72) Inventor: KAWASHIMA, Toshiyuki, Hanamaki-shi Iwate 025-0312 (JP)
(74) Representative: Klunker . Schmitt-Nilson . Hirsch
(86) International application number: PCT/JP2011/066984
(87) International publication number: WO 2012/017865

(57) **Abstract**

A three-way stopcock unit, a check valve, and a fluid substance transfer device, which are easily downsized, have a high yield, and are manufactured at low cost. The check valve in the three-way stopcock unit according to the present invention includes: a first housing having a valve seat in which an opening is provided; a second housing inserted into the first housing; and a valve element held between the valve seat of the first housing and an opening end portion of the second housing. The valve element has a substantially U-shaped cut line formed outside of an outer rim portion of the opening provided in the valve seat of the first housing, and inside of an inner rim portion of the second housing. A portion inside of the cut line is used as a switching valve, and the valve seat of the first housing and the opening end portion of the second housing are closed in a liquid-tight state at a portion outside of the cut line.

## Description

### Technical Field

The present invention relates to a three-way stopcock unit, a check valve provided in the three-way stopcock unit, and a fluid substance transfer device including the three-way stopcock unit.

### Background Art

There has been conventionally known a method of directly administering a fluid substance such as a nutritional composition to the stomach of a patient as a method for nourishing a patient who cannot take nutrition orally, for example.

In the method, a catheter is connected to the stomach of a patient, and a three-way stopcock unit is coupled to the catheter. A storage vessel that stores a fluid substance, and a syringe are further coupled to the three-way stopcock unit. The fluid substance stored in the storage vessel is temporarily taken out into the syringe, and supplied to the catheter from the syringe. The fluid substance is administered to a patient as described above.

For example, a device disclosed in Patent Literature 1 is known as a device for supplying a fluid substance. In the fluid substance transfer device in Patent Literature 1, a storage vessel such as a bag, a syringe, and a catheter are respectively connected to a three-way connector. In the device, a check valve is provided at a portion of the three-way connector connected with the catheter.

The check valve opens an opening of a valve seat upon receiving a pressure in one direction, and closes the opening upon receiving a pressure in an opposite direction.

For example, a structure disclosed in Patent Literature 2 is known as the structure of the check valve. The check valve in Patent Literature 2 is provided with a valve element that opens and closes an opening of a valve seat, and means (e.g., an O-ring) for maintaining the inside of the check valve in a liquid-tight state (that is, a close-contact state in which no fluid substance leaks).

### Citation List

### Patent Literature

Patent Literature 1: Japanese Patent Laid-Open No. 10-192395
Patent Literature 2: Japanese Patent Laid-Open No. 05-321824

### Summary of Invention

### Technical Problem

However, since the invention in Patent Literature 1 requires means for urging a valve element, means for supporting the valve element or the urging means, or the like, the structure of a portion in which a fluid substance flows becomes complicated. Meanwhile, in the check valve disclosed in Patent Literature 2, the valve element that opens and closes the opening of the valve seat, and the means (e.g., an O-ring) for maintaining the inside of the valve in a liquid-tight state are provided, and thus, the check valve has a complicated structure. Accordingly, in the devices in Patent Literatures 1 and 2, the check valve is difficult to downsize, the yield may be lowered, and the manufacturing cost may be increased.

Also, while a syringe is normally used when a substance such as semi-solid preparations is administered to a catheter from a vessel, there is a problem that the process takes much time. Moreover, while the substance is preferably treated in a closed system (that is, the vessel is preferably coupled to a three-way stopcock unit such that the fluid substance is not exposed to the outside air) from a hygienic standpoint, it is difficult for a conventional three-way stopcock unit to treat the fluid substance in a closed system.

The present invention has been made in view of the background art as described above, and it is an object thereof to provide a three-way stopcock unit, a check valve, and a fluid substance transfer device, which are easily downsized, have a high yield, are manufactured at low cost, and enable easy and hygienic administration of a substance such as semi-solid preparations through a closed system.

### Solution to Problem

A first aspect of the present invention provides a three-way stopcock unit comprising: a branch flow channel that is provided with a first flow channel, a second flow channel, and a third flow channel in which a fluid substance flows, the first flow channel, the second flow channel, and the third flow channel being joined together at one junction; a syringe that is provided at the third flow channel to supply a suction force for causing the fluid substance to flow into the first flow channel from outside, and an extrusion force for causing the fluid substance to flow outside of the second flow channel, to the branch flow channel; a first valve section that is provided at an opening end of the first flow channel to prevent the fluid substance from flowing out of the first flow channel; and a second valve section that is provided at an opening end of the second flow channel to prevent the fluid substance from flowing from the second flow channel; wherein at least one of the first valve section and the second valve section includes: a first housing having a first cylindrical portion, a valve seat that closes one of opening ends of the first cylindrical portion, and an opening that is provided in the valve seat; a second housing having a second cylindrical portion that is inserted into the first cylindrical portion of the first housing from another end of the opening ends; and a valve element that is formed of an elastic piece so as to be held between the valve seat of the first housing and an opening end portion of the cylindrical portion of the second housing; and wherein the valve element has a substantially U-shaped cut line formed outside of an outer rim portion of the opening provided in the valve seat of the first housing, and inside of an inner rim portion of the cylindrical portion of the second housing; and a portion inside of the cut line of the valve element is used as a switching valve, and the valve seat of the first housing and the cylindrical portion of the second housing are closed in a liquid-tight state at a portion outside of the cut line.

In the first aspect of the present invention, a cylinder section of the syringe and the third flow channel may be formed integrally with each other.

In the first aspect of the present invention, the first housing provided in the first valve section may be formed integrally with the first flow channel.

In the first aspect of the present invention, the second housing provided in the second valve section may be formed integrally with the second flow channel.

In the first aspect of the present invention, at least one of the first valve section and the second valve section may include a cylindrical connection member that is provided so as to be threadedly engaged or fitted with a connection section of the other element, and an engagement claw portion that is provided in the connection member to prevent disengagement of the other element by engagement with the other element when the connection member is connected to the other element.

In the first aspect of the present invention, the connection member may be formed as a female member into which the other element can be inserted, and the engagement claw portion may be provided projecting on an inner side surface of the connection member so as to form an acute angle in a direction in which the other element is inserted.

In the first aspect of the present invention, at least one of the first valve section and the second valve section may comprise; a configuration in which the first cylindrical portion is inserted into and threadedly engaged with the second cylindrical portion while the first cylindrical portion is being rotated in a predetermined direction by using a male thread formed on one of an inner side surface of the first cylindrical portion and an outer side surface of the second cylindrical portion, and a female thread formed in another surface of the inner side surface and the outer side surface, and a disengagement prevention mechanism that allows the rotation of the first cylindrical portion only in the predetermined direction, and thereby prevents disengagement of the second cylindrical portion from the first cylindrical portion.

In the first aspect of the present invention, the disengagement prevention mechanism may have one or a plurality of locking claw portions formed on one of the inner side surface of the first cylindrical portion and the outer side surface of the second cylindrical portion, and one or a plurality of locking convex portions formed on another surface of the inner side surface and the outer side surface, the locking claw portion being formed in an inclined state to a trailing side in the predetermined direction, and the locking convex portion allowing the rotation of the first cylindrical portion by pressing and deforming the locking claw portion in a direction substantially perpendicular to the rotation direction when the first cylindrical portion rotates in the predetermined direction, and restricting the rotation of the first cylindrical portion by engagement with the locking claw portion when the first cylindrical portion rotates in a direction opposite to the predetermined direction.

In the first aspect of the present invention, the plunger section of the syringe may include a substantially disk-shaped resin plunger head that slides within the cylinder section in a liquid-tight state, and a resin plunger rod that is formed integrally with the plunger head.

In the first aspect of the present invention, the plunger section of the syringe may include a plunger head that slides within the cylinder section, and a plunger rod that is connected to the plunger head.

In the first aspect of the present invention, the plunger section may further include a finger hooking section that is provided on a proximal end portion of the plunger rod to effect an axial movement operation of the plunger rod by a user's finger inserted therein, and two flange portions that are axially provided parallel to each other on the cylinder section of the syringe so as to have a distance therebetween into which a user's finger is inserted.

In the first aspect of the present invention, the finger hooking section may be a ring member that is provided projecting on the plunger rod.

In the first aspect of the present invention, the finger hooking section may include a belt-like body portion that is formed of a flexible material, and a hook portion that is provided at both ends of the body portion to maintain the finger hooking section by fitting into a groove formed in the proximal end portion of the plunger rod, the hook portion having the one or the plurality of narrow recessed portions, and one or a plurality of wide stopper portions that come into abutment against the proximal end portion from a lower side within the groove when the recessed portion is fitted into the groove, and the recessed portion and the stopper portion being alternately provided.

A second aspect of the present invention provides a check valve comprising: a first housing having a first cylindrical portion, a valve seat that closes one of opening ends of the first cylindrical portion, and an opening that is provided in the valve seat; a second housing having a second cylindrical portion that is inserted into the first cylindrical portion of the first housing from another end of the opening ends; and a valve element that is formed of an elastic piece so as to be held between the valve seat of the first housing and an opening end portion of the cylindrical portion of the second housing; wherein the valve element has a substantially U-shaped cut line formed outside of an outer rim portion of the opening provided in the valve seat of the first housing, and inside of an inner rim portion of the cylindrical portion of the second housing; and a portion inside of the cut line of the valve element is used as a switching valve, and the valve seat of the first housing and the cylindrical portion of the second housing are closed in a liquid-tight state at a portion outside of the cut line.

A third aspect of the present invention provides a fluid substance transfer device comprising: the three-way stopcock unit according to the above first aspect of the present invention; a storage vessel that stores a fluid substance flowing into the three-way stopcock unit from the first flow channel, and is connected to the second valve section; and a tube that is connected to the first valve section to supply the fluid substance into a human body.

### Advantageous Effects of Invention

In the present invention, the valve element is formed of an elastic body, and the switching valve is provided in a center portion of the valve seat. Therefore, the valve element can be elastically deformed to open and close the flow channel. Thus, in the first aspect of the present invention, it is not necessary to provide urging means for opening and closing the valve element, and a structure or the like for supporting the valve element or the urging means. As a result, in accordance with the first aspect of the present invention, a simplified and downsized structure can be obtained. Also, in the first aspect of the present invention, when the first cylindrical portion of the first housing is inserted into the second cylindrical portion of the second housing, the valve element is held between the valve seat of the first housing and the opening of the second housing. As a result, the switching valve of the valve element is opened when a fluid pressure is applied to the second housing from the first housing, and is closed when the fluid pressure is applied to the first housing from the second housing. Also, since the valve element is held between the valve seat of the first housing and the opening of the second housing, the valve seat and the opening are closed in a liquid-tight state. As described above, in accordance with the first aspect of the present invention, the valve element provided between the valve seat and the opening of the second cylindrical portion exerts both a function as a check valve and a function to maintain a joined portion between the first and second housings in a liquid-tight state at the same time. Accordingly, in accordance with the first aspect of the present invention, the check valve can be easily downsized, the yield of the check valve can be improved, and the manufacturing cost can be kept low.

In the first aspect of the present invention, the cylinder section of the syringe and the third flow channel are formed integrally with each other, so that the manufacturing cost is reduced, the assembling operation is simplified, and liquid leakage between the cylinder section and the third flow channel can be reliably prevented.

In the first aspect of the present invention, the first housing and the first flow channel are formed integrally with each other, so that the manufacturing cost can be lowered, the assembling operation can be simplified, and liquid leakage between the first housing and the first flow channel can be reliably prevented.

In the first aspect of the present invention, the second housing and the second flow channel are formed integrally with each other, so that the manufacturing cost can be lowered, the assembling operation can be simplified, and liquid leakage between the second housing and the second flow channel can be reliably prevented.

In the first aspect of the present invention, the disengagement prevention mechanism that prevents the disengagement of the other element is provided in the cylindrical connection member threadedly engaged or fitted with the other element, so that a trouble that the other element falls off by accident can be reliably prevented. For example, when the other element is a storage vessel for a fluid substance, it is made impossible to remove the storage vessel from the three-way stopcock unit and connect a new storage vessel thereto after the fluid substance in the storage vessel is used up. For example, in a case in which the fluid substance is administered into the body of a patient, the three-way stopcock unit needs to be replaced each time the storage vessel becomes empty from a hygienic standpoint in some cases. In such cases, a user can be prevented from using the three-way stopcock unit a plurality of times by mistake in accordance with the first aspect of the present invention.

Also, the locking claw portion and the locking convex portion are used to constitute the disengagement prevention mechanism, so that the highly reliable disengagement prevention mechanism can be obtained at low cost.

In the first aspect of the present invention, the plunger head is formed so as to slide within the cylinder section in a liquid-tight state, and the plunger head and the plunger rod are formed integrally with each other, so that the number of components can be reduced, and the manufacturing cost can be thereby reduced.

In the first aspect of the present invention, the finger hooking section is provided on the proximal end portion of the plunger rod, and the two flanges are provided on the cylinder section, so that an operator can push and pull the plunger section of the syringe by only one hand while inserting the thumb into the finger hooking section and inserting the index finger and the middle finger of the hand between the two flange portions to grip the three-way stopcock unit. Accordingly, the three-way stopcock unit with good operability can be provided.

In accordance with the second aspect of the present invention, the check valve that is downsized, has a high yield, and is manufactured at low cost can be provided because of the same reasons as those of the above first aspect of the present invention.

In accordance with the third invention, the fluid substance transfer device that is downsized, has a high yield, and is manufactured at low cost can be provided because of the same reasons as those of the above first aspect of the present invention.

### Brief Description of Drawings

[Figure 1] Figure 1 is a conceptual view illustrating an entire configuration of a fluid substance transfer device according to Embodiment 1.
[Figure 2] Figure 2 is an exploded sectional view of a three-way stopcock unit according to Embodiment 1.
[Figure 3] Figure 3 is a front view of a connection member of the three-way stopcock unit according to Embodiment 1.
[Figure 4] Figures 4 are views of a valve element of the three-way stopcock unit according to Embodiment 1: (a) is a front view; and (b) is a side view.
[Figure 5] Figure 5 is a sectional view illustrating a state before a bag and a transfer tube are connected to the three-way stopcock unit according to Embodiment 1.
[Figure 6] Figure 6 is a sectional view illustrating a state in which the bag and the transfer tube are connected to the three-way stopcock unit according to Embodiment 1.
[Figure 7] Figure 7 is a sectional view illustrating a fluid substance transfer device according to Embodiment 2 of the invention.
[Figure 8] Figure 8 is a sectional view illustrating a state before a bag and a transfer tube are connected to a three-way stopcock unit of a fluid substance transfer device according to Embodiment 3 of the invention.
[Figure 9] Figure 9 is a sectional view illustrating a state in which the bag and the transfer tube are connected to the three-way stopcock unit of the fluid substance transfer device according to Embodiment 3.
[Figure 10] Figure 10 is a sectional view illustrating a state before a bag and a transfer tube are connected to a three-way stopcock unit of a fluid substance transfer device according to Embodiment 4.
[Figure 11] Figure 11 is a sectional view illustrating a state in which the bag and the transfer tube are connected to the three-way stopcock unit of the fluid substance transfer device according to Embodiment 4.
[Figure 12] Figure 12 is a conceptual view illustrating an entire configuration of a fluid substance transfer device according to Embodiment 5.
[Figure 13] Figure 13 is an exploded sectional view of a three-way stopcock unit according to Embodiment 5.
[Figure 14] Figures 14 are views illustrating the configuration of a disengagement prevention mechanism according to Embodiment 5: (a) is a sectional view of a locking claw portion; and (b) is a sectional view of a locking convex portion.
[Figure 15] Figures 15 are views illustrating the operation of the disengagement prevention mechanism according to Embodiment 5: (a) is a conceptual view in engagement; and (b) is a conceptual view in disengagement.
[Figure 16] Figure 16 is a perspective view conceptually illustrating the structure of a finger hooking section according to Embodiment 5.

### Description of Embodiments

### [Embodiment 1]

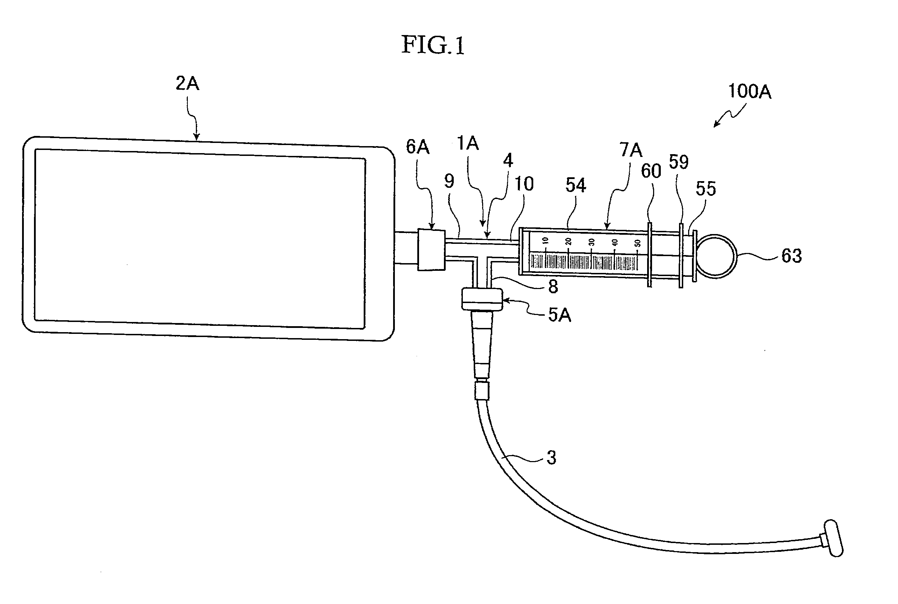

Figures 1 to 6 show Embodiment 1 of the present invention.

A fluid substance transfer device 100A according to Embodiment 1 of the present invention is used for transferring a fluid substance stored in a bag 2A to an indwelling catheter in a patient. That is, a nutritional composition in the form of a liquid, a gel, a sol, or a mixture of one of them and a solid is administered to a part of a patient (not shown) by use of the fluid substance transfer device 100A.

The fluid substance transfer device 100A includes a three-way stopcock unit 1A, the bag 2A that stores a fluid substance, and a transfer tube 3 that transfers the fluid substance into a human body.

The three-way stopcock unit 1A is formed of a material with rigidity and liquid tightness, e.g., a resin such as polypropylene. The three-way stopcock unit 1A includes a branch flow channel 4, a first valve section 5A, a second valve section 6A, and a syringe 7A, and functions as a so-called three-way valve.

In the branch flow channel 4, a first flow channel 8, a second flow channel 9, and a third flow channel 10 are joined together at one junction 11. The flow channels 8, 9, and 10 are respectively configured such that a fluid can flow therein. While each of the flow channels 8, 9, and 10 is formed with a length of, for example, about 0.6 to 1.0 cm in Embodiment 1, the flow channels may have a length longer or shorter than the above lengths.

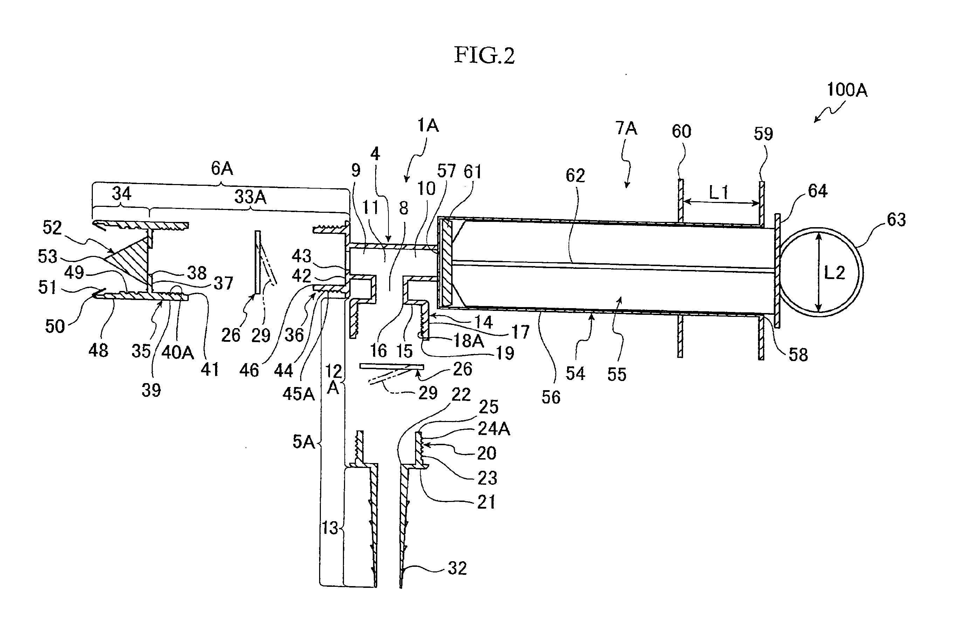

The first valve section 5A is arranged at an end portion of the first flow channel 8 of the branch flow channel 4. As shown in Figure 2, the first valve section 5A includes a first check valve section 12A as a "check valve," and a coupling section 13.

The first check valve section 12A includes a first housing 14, a second housing 20, and a valve element 26.

The first housing 14 is provided at the end portion of the first flow channel 8, and is formed integrally with the first flow channel 8. The first housing 14 includes a substantially disk-shaped valve seat 15, and a first cylindrical portion 17. A flow inlet 16 as an "opening" in communication with the first flow channel 8 is formed in a substantially center portion of the valve seat 15. The valve element described later is disposed on the valve seat 15. A rim portion of the valve seat 15 is joined to one end of the first cylindrical portion 17. The first cylindrical portion 17 having a substantially cylindrical shape is arranged in an extended direction from the first flow channel 8. A female thread 18A is formed in an inner circumference of the first cylindrical portion 17. A substantially circular opening 19 is formed on the other end side of the first cylindrical portion 17.

The second housing 20 is formed of the same material as the first housing 14. The second housing 20 is fabricated so as to be attachable and detachable to and from the first housing 14. A substantially disk-shaped intermediate wall portion 21 is provided on one end side of the second housing 20. Moreover, a flow outlet 22 in communication with the coupling section described later is formed in a substantially center portion of the intermediate wall portion 21. The intermediate wall portion 21 is provided on one end side of a second cylindrical portion 23. The second cylindrical portion 23 has a substantially cylindrical shape. An outer diameter of the second cylindrical portion 23 is substantially the same as an inner diameter of the first cylindrical portion 17. A male thread 24A is provided on an outer circumferential surface of the second cylindrical portion 23 for threaded engagement with the female thread 18A. A substantially circular opening 25 is formed on the other end side of the second cylindrical portion 23.

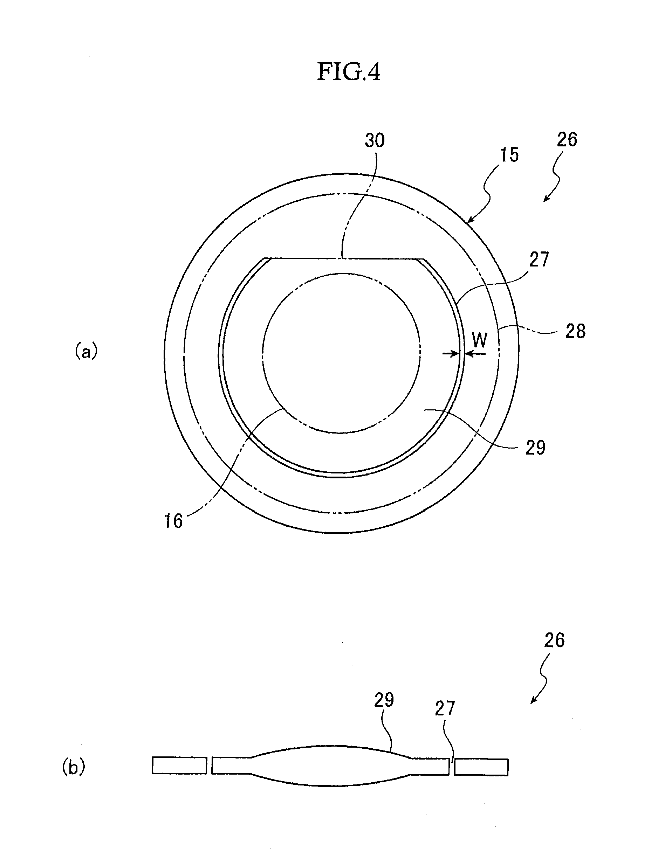

The valve element 26 shown in Figures 4(a) and (b) is formed of an elastic body with liquid tightness such as silicon. The valve element 26 has a substantially disk shape as an entire shape.

A substantially U-shaped cut line 27 is provided in the valve element 26. As shown in Figures 4, the cut line 27 is formed in a slit shape with a gap W. The valve element 26 is held between the valve seat 15 and the opening 25 of the second housing 20. As shown in Figures 4, the cut line 27 is arranged inside of a position in abutment with an inner rim portion 28 of the opening 25, and is also arranged outside of the position of the flow inlet 16 in the valve seat 15.
A portion inside of the cut line 27 functions as a switching valve 29. That is, when the valve element 26 is disposed on the valve seat 15, the switching valve 29 moves in a rotation direction about a virtual line 30 (see Figures 4) that connects both end portions of the cut line 27, to open and close the flow inlet 16. As shown in Figure 4(b), the switching valve 29 is formed so as to be thick around its center, and be thin around its rim portion in the present embodiment. It is thus possible to prevent the switching valve 29 from being bent to cause malfunction when the switching valve 29 is opened or closed. When a plurality of valve elements 26 are washed at the same time after manufacture, the valve elements 26 can also be prevented from sticking to each other.
The valve element 26 has substantially the same diameter as the inner diameter of the first housing 14, e.g., a diameter of about 1.5 cm. Also, a thickness of the valve element 26 is, for example, about 1.0 mm at the rim portion, and about 2.0 mm at a thickest portion of the switching valve 29. Note that the diameter and the thickness of the valve element 26 may be larger or smaller than the above dimensions.
A valve element having a substantially uniform thickness may also be used instead of the valve element 26 in Figure 4(b).

The coupling section 13 is provided projecting on an opposite side to the intermediate wall portion 21 of the second housing 20. The second housing 20 and the coupling section 13 are formed integrally with each other. The coupling section 13 has a substantially cylindrical shape such that its outer diameter is reduced toward a distal end. A plurality of (e.g., four) anti-slips 32 having a substantially wedge shape in section are provided projecting on an outer circumferential surface of the coupling section 13. The anti-slips 32 are circularly arranged along the outer circumference of the coupling section 13. As long as the anti-slips 32 can appropriately prevent the transfer tube 3 coupled to the coupling section 13 from falling off, the anti-slips 32 may have a sectional shape other than the wedge shape. The anti-slips 32 may not necessarily be arranged circularly, but may be provided only in a portion of a radial direction.

The second valve section 6A is provided at an end portion of the second flow channel 9 of the branch flow channel 4. The second valve section 6A includes a second check valve section 33A as a "check valve," and a connection section 34 as a "connection member."

The second check valve section 33A includes a first housing 35, a second housing 36, and a valve element 26.

The first housing 35 and the connection section 34 are formed integrally with each other by using the same material as the branch flow channel 4. A substantially disk-shaped valve seat 37 is provided at one end of the first housing 35. Moreover, a flow inlet 38 as an "opening" in communication with the connection section 34 is formed in a substantially center portion of the valve seat 37. The first housing 35 includes a first cylindrical portion 39, a female thread 40A, and an opening 41. The structures, dimensions, and materials of the respective components are the same as those of the first cylindrical portion 17, the female thread 18A, and the opening 19 described above.

The second housing 36 is provided at the end portion of the second flow channel 9, and is formed integrally with the second flow channel 9. The second housing 36 includes an intermediate wall portion 42, a flow outlet 43, a second cylindrical portion 44, a male thread 45A, and an opening 46. The intermediate wall portion 42 has a substantially disk shape. The flow outlet 43 is provided in a substantially center portion of the intermediate wall portion 42. The flow port 43 communicates with the second flow channel 9. The structures, dimensions, and materials of the second cylindrical portion 44, the male thread 45A, and the opening 46 are the same as those of the second cylindrical portion 23, the male thread 24A, and the opening 25 described above.

The structure, dimension, and material of the valve element 26 are the same as those of the valve element 26 disposed in the first check valve section 12A. When the valve element 26 is disposed on the valve seat 37, a switching valve 29 moves in a rotation direction about a virtual line 30, to open and close the flow inlet 38.

The connection section 34 is disposed on the valve seat 37 on an opposite side to the first housing 35. The connection section 34 is a female member into which a discharge section (described later) of the bag 2A can be inserted. The connection section 34 includes a cylindrical portion 48, a female thread 49, an opening 50, an engagement claw portion 51, and a projection portion 52.

The cylindrical portion 48 is arranged on an extended line of the first cylindrical portion 39 provided in the first housing 35, and is formed in the same cylindrical shape as the first cylindrical portion 39. The female thread 49 is provided in an inner circumference of the cylindrical portion 48. The substantially circular opening 50 is formed at a distal end of the cylindrical portion 48.

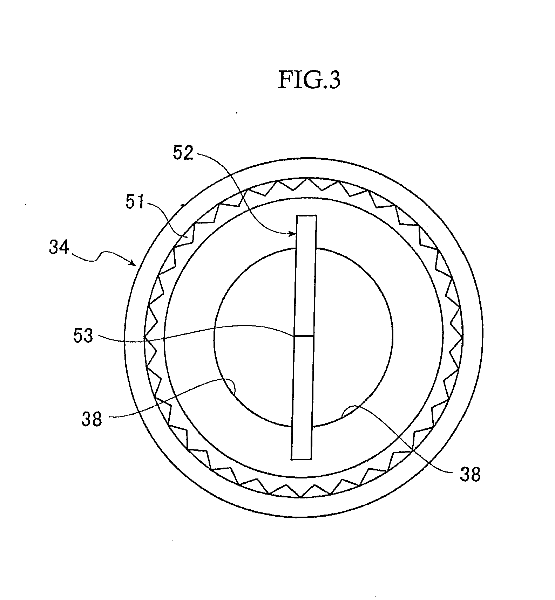

The engagement claw portion 51 is provided at the opening 50. As shown in Figure 3, the engagement claw portion 51 is composed of a plurality of substantially triangular projections, which are provided projecting inward so as to form an acute angle toward the flow inlet 38 (i.e., a direction in which the bag 2A is inserted) as shown in Fig. 2. As described later, the engagement claw portion 51 has a function to prevent disengagement of the bag 2A from the connection section 34 by engagement with the discharge section (described later) of the bag 2A when the discharge section (described later) is inserted into the connection section 34.

The projection portion 52 is formed in a substantially triangular plate-like shape as shown in Figures 2 and 3. The projection portion 52 is provided projecting on a back side of the valve seat 37 such that a plate surface is directed substantially perpendicular to the valve seat 37, and a distal end portion 53 is directed toward the opening 50.

The syringe 7A has a cylinder section 54 and a plunger section 55 that is inserted into the cylinder section 54.

The cylinder section 54 is formed integrally with the third flow channel 10 at an end portion of the third flow channel 10. The cylinder section 54 has a cylinder body portion 56, a flow inlet and outlet 57, an opening end portion 58, a first flange portion 59, and a second flange portion 60.

The cylinder body portion 56 is formed in a substantially cylindrical shape. In Embodiment 1, the cylinder body portion 56 is formed with a length of about 9 to 10 cm, and a diameter of about 2 cm. Note that the length and the diameter may be larger or shorter than the above dimensions.

The flow inlet and outlet 57 is formed on one end side of the cylinder section 54 so as to communicate with the third flow channel 10. The opening end portion 58 is formed on the other end side of the cylinder body portion 56. The opening end portion 58 has substantially the same diameter as an inner diameter of the cylinder body portion 56. The first flange portion 59 is provided projecting radially outward from the opening end portion 58. The second flange portion 60 is provided parallel to the first flange portion 59 at a position closer to a center from the first flange portion 59. A distance L1 between the first flange portion 59 and the second flange portion 60 shown in Figure 2 is set to a length with which the syringe 7A can be operated by inserting a user's fingers (e.g., the index finger and the middle finger) therein, e.g., about 2 to 3 cm.

The plunger section 55 is formed integrally by using the same material as the cylinder section 54. The plunger section 55 has a plunger head 61, a plunger rod 62, and a ring member 63 as a "finger hooking section." The ring member 63 is provided on a proximal end portion 64 of the plunger rod 62.

The plunger head 61 is formed in a disk shape having substantially the same diameter as that of an inner circumference of the cylinder body portion 56. No gasket is provided at the plunger head 61. However, when sliding along the inner circumferential portion of the cylinder body portion 56, the plunger head 61 comes into close contact with an inner wall surface of the cylinder body portion 56. Accordingly, high liquid tightness is obtained in an abutment portion between the plunger head 61 and the cylinder body portion 56.

The plunger rod 62 is formed in an elongated rod-like shape with a substantially cross-shaped section. The plunger rod 62 has an entire length larger than an entire length of the cylinder body portion 56. Thus, when the plunger head 61 is inserted to a backmost position of the cylinder body portion 56, the ring member 63 on the proximal end 64 is exposed outside from the cylinder body portion 56. An inner diameter L2 of the ring member 63 shown in Figure 2 is set to a length with which the syringe 7A can be operated by inserting a user's finger (e.g. the thumb) therein, e.g., a diameter of about 2 to 3 cm.

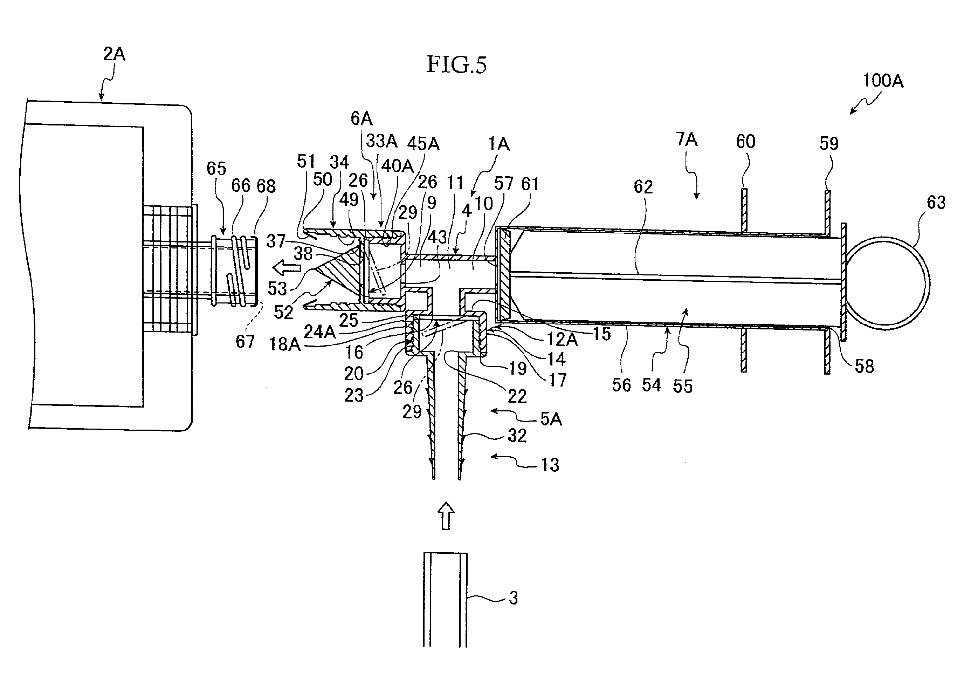

Any vessel in which a fluid substance is stored can be used as the bag 2A as a "storage vessel." As shown in Figure 5, a resin flexible vessel is used as the bag 2A in Embodiment 1. The cylindrical discharge section 65 as "another element" is provided at one end portion of the bag 2A. The discharge section 65 is configured to be coupled to the connection section 34 of the second valve section 6A in a liquid-tight manner. A male thread 66 is formed on an outer circumferential surface of the discharge section 65 for threaded engagement with the female thread 49. An opening 67 at an end portion of the discharge section 65 is sealed by a seal member 68. An aluminum foil or other film-like material which can prevent oxidization or erosion of the fluid substance stored in the bag 2A is used as the seal member 68.

Next, an assembling procedure for the fluid substance transfer device 100A according to Embodiment 1 will be described.

When the fluid substance transfer device 100A shown in Figure 2 is assembled together, a user places the valve element 26 on the valve seat 15 of the first housing 14 first, and threadedly engages the male thread 24A of the second housing 20 with the female thread 18A, to assemble the first valve section 5A together. Subsequently, the user places the valve element 26 on the valve seat 37 of the first housing 35, and threadedly engages the male thread 45A of the second housing 36 with the female thread 40A, to assemble the second valve section 6A together. At this time, as shown in Figure 5, the valve element 26 of the first valve section 5A is held between the valve seat 15 and the opening 25. Similarly, the valve element 26 of the second valve section 6A is held between the valve seat 37 and the opening 46. When the threaded engagement states between the female threads 18A and 40A and the male threads 24A and 45A are adjusted, the valve element holding states can be freely adjusted, thereby reliably preventing liquid leakage of the fluid substance.

The user further inserts the plunger section 55 into the cylinder section 54, to complete the syringe 7A.

Next, an operational procedure for the fluid substance transfer device 100A according to the present embodiment will be described.

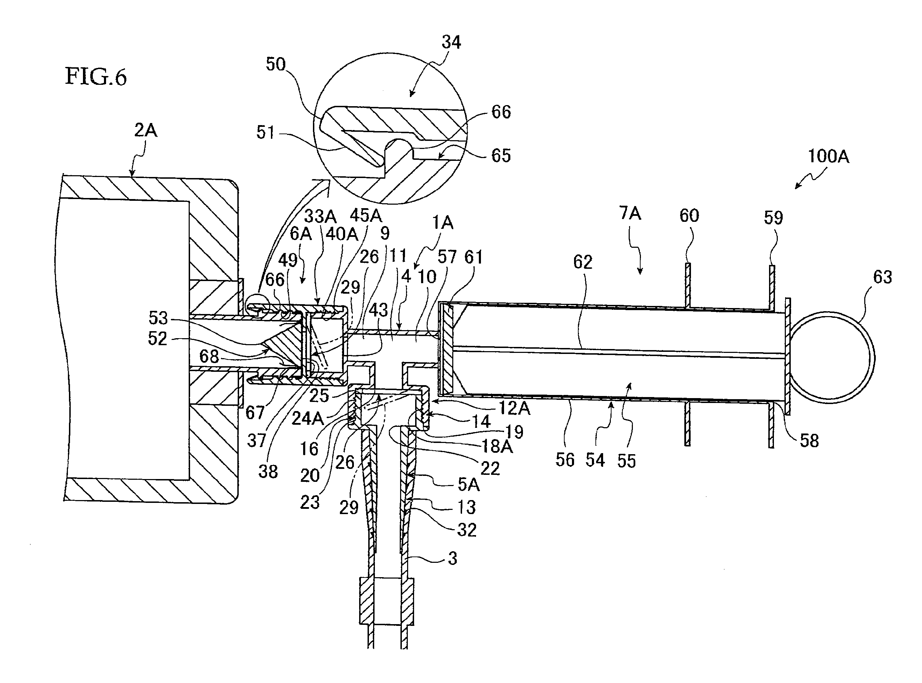

As shown in Figure 5A, a user brings the discharge section 65 of the bag 2A in which the fluid substance is stored into abutment against the connection section 34 of the second valve section 6A, and rotates the discharge section 65. The female thread 49 and the male thread 66 are thereby threadedly engaged together to cause the distal end portion 53 of the projection portion 52 to approach and break through the seal member 68. As a result, the opening 67 is exposed. When the female thread 49 and the male thread 66 are further threadedly engaged together to completely connect the discharge section 65 and the second valve section 6A together, a distal end of the engagement claw portion 51 provided at the connection section 34 becomes engaged with an end portion of the male thread 66 as shown in Figure 6. As a result, the male thread 66 cannot be rotated in a direction in which the threaded engagement is released. That is, once the bag 2A is connected to the three-way stopcock unit 1A, the bag 2A and the three-way stopcock unit 1A cannot be separated from each other anymore. It is thus possible to reliably avoid a trouble that another element engaged with the three-way stopcock unit 1A falls off by accident.

Subsequently, the user inserts the coupling section 13 of the first valve section 5A into one end of the transfer tube 3. The transfer tube 3 is thereby coupled to the coupling section 13 as shown in Figure 6. In the above state, a strong frictional force is applied to an inner wall of the transfer tube 3 from the anti-slips 32 of the coupling section 13, so that the transfer tube 3 is not easily separated from the coupling section 13.

The other end portion of the transfer tube 3 is coupled to an indwelling catheter (not shown) in a patient in the above state.

After that, by operating the syringe 7A, the user administers the fluid substance into the body of the patient. To be more specific, the user pulls the plunger rod 62 in a direction in which the plunger head 61 moves away from the flow inlet and outlet 57 first. A pressure within the branch flow channel 4 thereby falls below the atmospheric pressure. Thus, the switching valve 29 of the first check valve section 12A is closed to close the flow inlet 16. Also, the switching valve 29 of the second check valve section 33A is opened to open the flow inlet 38. As a result, the fluid substance is sucked out from inside the bag 2A, and transferred into the cylinder section 54 of the syringe 7A through the first flow channel 8 and the third flow channel 10.

Subsequently, the user pushes the plunger rod 62 in a direction in which the plunger head 61 approaches the flow inlet and outlet 57. The pressure within the branch flow channel 4 thereby rises above the atmospheric pressure. Thus, the switching valve 29 of the second check valve section 33A is closed to close the flow inlet 38. Also, the switching valve 29 of the first check valve section 12A is opened to open the flow inlet 16. As a result, the fluid substance is pushed out from the cylinder section 54, and supplied into the body of the patient through the third flow channel 10, the second flow channel 9, the second valve section 6A, and the transfer tube 3.

When the plunger head 61 moves in the suction direction and the extrusion direction, the plunger head 61 slides within the cylinder section 54 in a liquid-tight state. Thus, there occurs no liquid leakage between the plunger head 61 and the cylinder section 54.

To move the plunger head 61, the user inserts two fingers of one hand (e.g., the index finger and the middle finger of the right hand) between the first flange portion 59 and the second flange portion 60 of the syringe 7A, and grips the cylinder section 54 with the fingers. The user further inserts another finger of the same hand (e.g., the thumb of the right hand) into the ring member 63 of the plunger section 55. By pulling or pushing the plunger section 55 in the above state, the user can grip and operate the syringe 7A by only one hand. That is, in accordance with Embodiment 1, the three-way stopcock unit 1A and the fluid substance transfer device 100A with good operability can be provided.

As shown in Figures 4, the gap W is formed in the cut line 27 of the valve element 26. It is thus possible to avoid a trouble that, when the switching valve 29 is closed, the switching valve 29 overlaps with a peripheral portion of the valve element 26, so that the flow inlets 16 and 38 are not closed in a liquid-tight manner.

The transfer tube 3 is pulled out of the coupling section 13 after use of the fluid substance transfer device 100A. The three-way stopcock unit 1A and the bag 2A are discarded in a state in which the three-way stopcock unit 1A and the bag 2A are connected together. Since the engagement claw portion 51 of the connection section 34 is engaged with the male thread 66 of the bag 2A as described above, the bag 2A cannot be separated from the three-way stopcock unit 1A.

In a case in which the fluid substance transfer device 100A is used in dietary intake of a patient, it is desirable from a hygienic standpoint that the fluid substance transfer device 100A be prohibited from being used a plurality of times, and be discarded each time the bag 2A becomes empty. To this end, the coupling section 13 or the discharge section 65 needs to be prevented from being disengaged once the coupling section 13 or the discharge section 65 is mounted to the branch flow channel 4. In Embodiment 1, since the engagement claw portion 51 or the like is used, the disengagement of the coupling section 13 or the discharge section 65 can be reliably prevented with a simple and inexpensive structure. Accordingly, in accordance with Embodiment 1, it is possible to reliably avoid a problem that the three-way stopcock unit 1A is reused without being discarded, and a patient can be kept in good hygiene.

As described above, in Embodiment 1, when the plunger section 55 of the syringe 7A is pulled, the first valve section 5A is closed, and the second valve section 6A is opened. Meanwhile, when the plunger section 55 is pushed, the first valve section 5A is opened, and the second valve section 6A is closed. Therefore, by connecting the first valve section 5A to the transfer tube 3, and the second valve section 6A to the bag 2A, the fluid substance can be administered into the body of a patient.

In Embodiment 1, the switching valves 29 are fabricated by providing the substantially U-shaped cut lines in the elastic valve elements 26, and the switching valves 29 are used to open and close the flow inlets 16 and 38 provided in the center portions of the valve seats 15 and 37. The switching valves 29 are elastically deformed to open and close the flow inlets 16 and 38. Accordingly, in accordance with Embodiment 1, it is not necessary to provide urging means for opening and closing the valve elements 26, and a structure or the like for supporting the valve elements 26 and the urging means. Because of the above reasons, in accordance with Embodiment 1, the three-way stopcock unit 1A can be simplified in structure and downsizing.

Also, in Embodiment 1, the valve elements 26 are held between the valve seats 15 and 37 of the first housings 14 and 35, and the openings 25 and 41 of the second housings 20 and 36. As a result, the switching valves 29 of the valve elements 26 are opened when a fluid pressure is applied to the second housings 20 and 36 from the first housings 14 and 35, and are closed when the fluid pressure is applied to the first housings 14 and 35 from the second housings 20 and 36. Also, since the valve elements 26 are held between the valve seats 15 and 37 of the first housings 14 and 35, and the openings 25 and 41 of the second housings 20 and 36, the valve seats 15 and 37 and the openings 25 and 41 are closed in a liquid-tight state. As described above, in Embodiment 1, the valve elements 26 provided between the valve seats 15 and 37, and the openings 25 and 41 exert both the function as the check valve and the function to maintain a joined portion between the first and second housings in a liquid-tight state at the same time. Accordingly, in accordance with Embodiment 1, the check valve can be easily downsized, the yield of the check valve can be improved, and the manufacturing cost can be saved.

In Embodiment 1, the branch flow channel 4, the cylinder section 54 of the syringe 7A, the first housing 14 of the first valve section 5A, and the second housing 36 of the second valve section 6A are formed integrally with each other. Accordingly, in accordance with Embodiment 1, the manufacturing cost can be lowered, and the assembling operation can be also simplified. Also, in accordance with Embodiment 1, liquid leakage between the cylinder section 54 and the branch flow channel 4, between the first valve section 5A and the branch flow channel 4, and between the second valve section 6A and the branch flow channel 4 can be reliably prevented.

In Embodiment 1, the plunger head 61 and the plunger rod 62 are provided in the plunger section 55 of the syringe 7A, and the plunger head 61 is formed of a substantially disk-shaped resin so as to slide within the cylinder section 54 in a liquid-tight state. The liquid tightness can be thereby ensured without providing a gasket at the plunger head 61. As a result, the plunger section 55 can be formed as one integrated component. Therefore, the three-way stopcock unit 1A with no liquid leakage from the plunger section 55 can be manufactured at low cost.

In Embodiment 1, the connection section 34 is formed as a female member into which the discharge section 65 of the bag 2A can be inserted. In Embodiment 1, the engagement claw portion 51 is also formed so as to project from inside the opening 50 of the connection section 34. Accordingly, in accordance with Embodiment 1, the engagement claw portion 51 can reliably prevent disconnection of the discharge section 65.

In Embodiment 1, the ring member 63 as the finger hooking section is provided projecting from the plunger rod 62. The operability of the three-way stopcock unit 1A and the fluid substance transfer device 100A can be thereby improved.

Since the fluid substance transfer device 100A according to Embodiment 1 is used by connecting the bag 2A and the transfer tube 3 to the three-way stopcock unit 1A according to Embodiment 1, the fluid substance transfer device 100A can be easily downsized, has a high yield, and can be manufactured at low cost.

### [Embodiment 2]

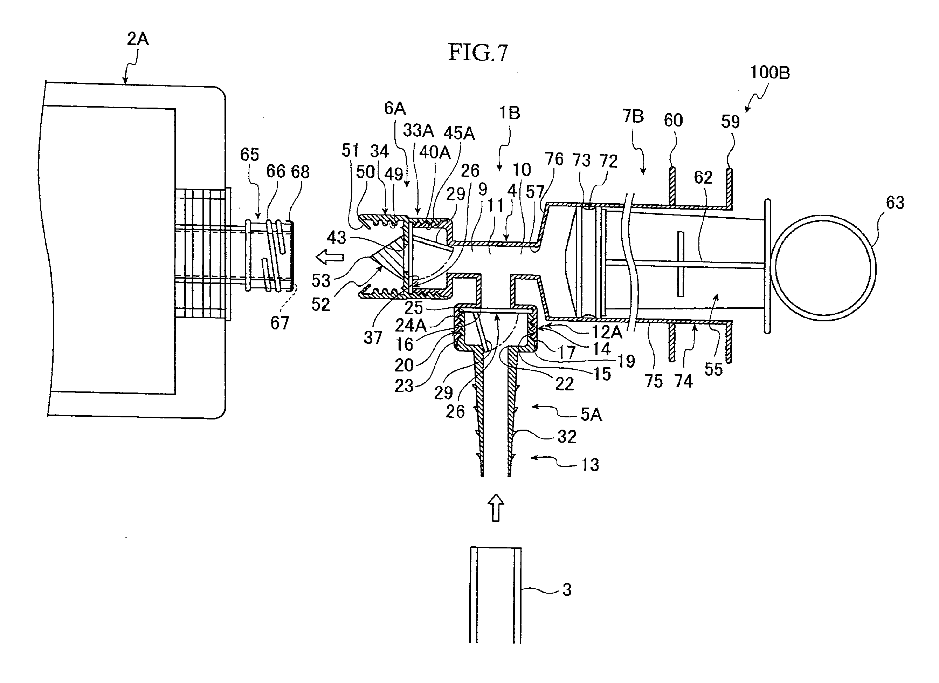

Figure 7 shows Embodiment 2 of the present invention.

In a fluid substance transfer device 100B according to Embodiment 2, a syringe 7B provided in a three-way stopcock unit 1B has a different configuration from the syringe 7A of Embodiment 1. In the syringe 7B of Embodiment 2, a gasket 73 is provided at a plunger head 72 of a plunger section 71. Also, one end portion 76 of a cylinder body portion 75 is formed into a shape substantially corresponding to an end portion shape of the plunger head 72. The other elements are the same as those of Embodiment 1.

In accordance with Embodiment 2, since the gasket 73 is provided at the plunger head 72, liquid tightness between an inner portion of a cylinder section 74 and the plunger section 71 can be easily ensured, and it is thus not necessary to fabricate the plunger head 72 with high accuracy.

### [Embodiment 3]

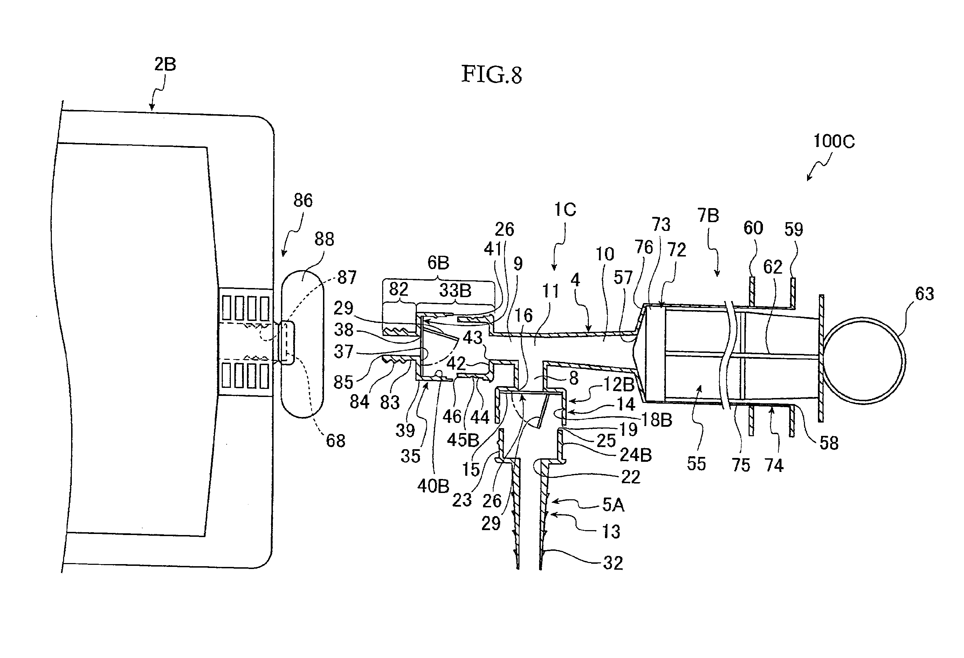

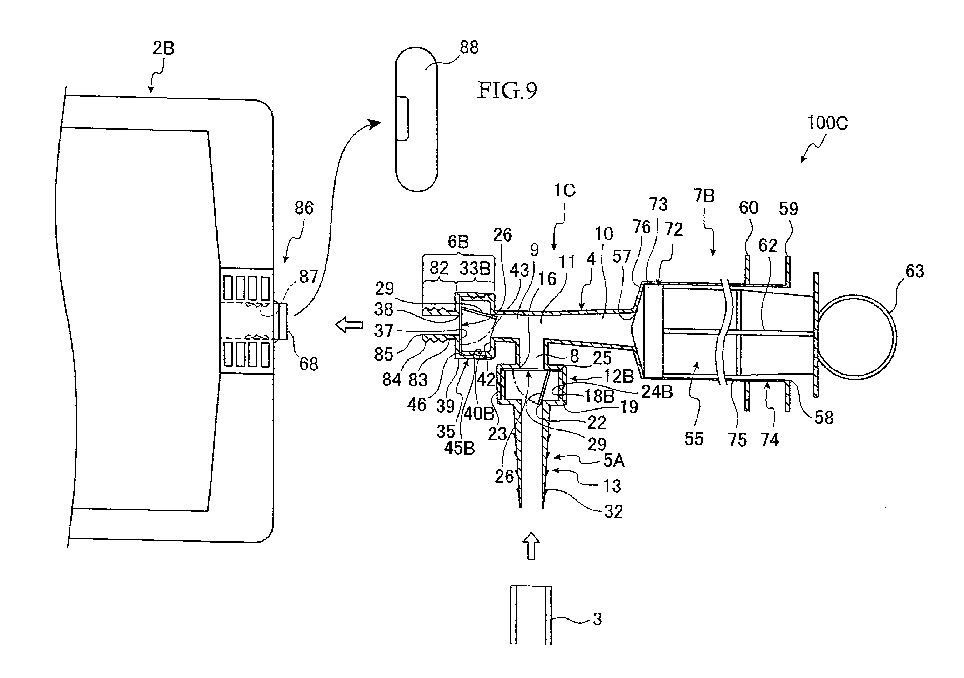

Figures 8 and 9 show Embodiment 3 of the present invention.

As shown in Figure 8, a fluid substance transfer device 100C according to Embodiment 3 includes a second valve section 6B and a bag 2B instead of the second valve section 6A and the bag 2A of Embodiments 1 and 2 described above. A connection section 82 and a discharge section 86 are respectively provided in the second valve section 6A and the bag 2B.

The connection section 82 is a male member which is inserted into the bag 2B. The connection section 82 includes a cylindrical portion 83. A male thread 84 is formed on an outer circumferential surface of the cylindrical portion 83. An opening 85 is also provided at a distal end of the cylindrical portion 83.

The discharge section 86 of the bag 2B includes a female thread 87 provided in an inner circumferential surface. The female thread 87 is formed so as to be threadedly engaged with the male thread 84. A resin cap member 88 is disposed on an outer side of the discharge section 86.

The fluid substance transfer device 100C according to Embodiment 3 also includes a first check valve section 12B and a second check valve section 33B instead of the first check valve section 12A and the second check valve section 33A according to Embodiments 1 and 2 described above. The first check valve section 12B and the second check valve section 33B respectively include concave groove-like fitted portions 18B and 45B, and projection-like fitting portions 24B and 40B instead of the female threads 18A and 40A, and the male threads 24A and 45A according to Embodiments 1 and 2 described above. The other elements are substantially the same as those of Embodiment 2.

As shown in Figure 9, when the first check valve section 12B and the second check valve section 33B are respectively assembled together, the fitted portion 18B and the fitting portion 24B are fitted together, and the fitted portion 40B and the fitting portion 45B are fitted together. When the connection section 82 and the discharge section 65 are connected together, the cap member 88 of the bag 2B is bent and removed, and the male thread 84 and the female thread 87 are threadedly engaged together. The seal member 68 of the bag 2B is thereby torn, so that the discharge section 86 and the second valve section 6B can be connected together. The other operations are substantially the same as those of Embodiment 1.

Although a syringe in Embodiment 3 has the same configuration as that of Embodiment 2 described above, the same syringe as the syringe 7A of Embodiment 1 described above may also be employed.

The second valve section 6B and the bag 2B according to Embodiment 3 may be applied to the three-way stopcock unit and the fluid substance transfer device having the female threads 18A and 40A, and the male threads 24A and 45A as in Embodiments 1 and 2 described above.

Also, the fitted portions 18B and 45B, and the fitting portions 24B and 40B according to Embodiment 3 may be applied to the three-way stopcock unit and the fluid substance transfer device having the second valve section 6A and the bag 2A as in Embodiments 1 and 2 described above.

### [Embodiment 4]

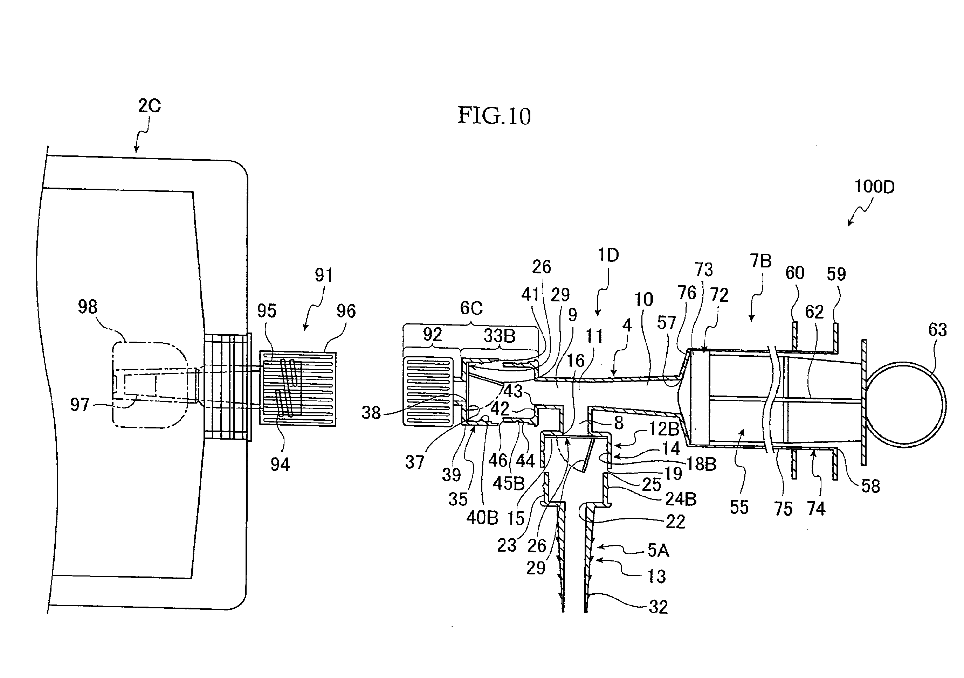

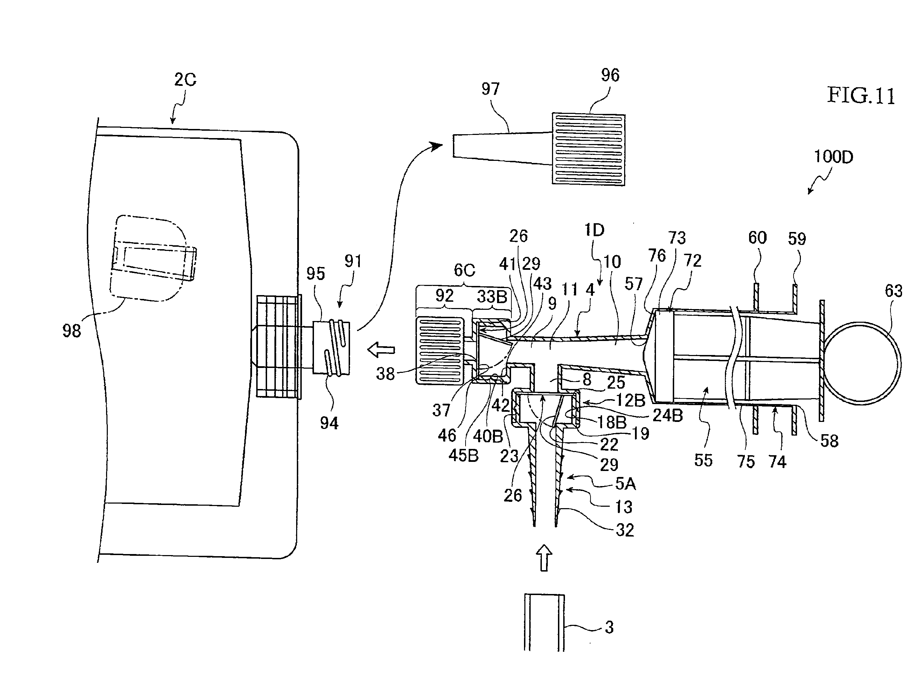

Figures 10 and 11 show Embodiment 4 of the present invention.

As shown in Figure 10, a fluid substance transfer device 100D according to Embodiment 4 includes a second valve section 6C and a bag 2C instead of the second valve sections 6A and 6B, and the bags 2A and 2B of Embodiments 1 to 3. A connection section 92 and a discharge section 91 are respectively provided in the second valve section 6A and the bag 2C.

The connection section 92 is an axially rotatable female member. A female thread (not shown) is formed in an inner circumferential surface of a cylindrical body portion of the connection section 92.

The discharge section 91 has a discharge body portion 95 having a cylindrical shape. A male thread 94 is formed on an outer circumferential surface of the discharge body portion 95. The male thread 94 is threadedly engaged with the unillustrated female thread. Moreover, the outside of the discharge body portion 95 is covered with a resin cap member 96. The cap member 96 includes a distal end portion 97. The distal end portion 97 is inserted into the discharge body portion 95. A resin distal end protection section 98 is provided inside the bag 2C adjacent to the discharge body portion 95.

The other elements are the same as those of Embodiment 3.

As shown in Figure 11, when the connection section 92 and the discharge section 91 are connected together, the distal end protection section 98 is bent from outside the bag 2C, and thereby separated from the discharge body portion 95 first. The cap member 96 can be thereby separated from the discharge body portion 95. After the cap member 96 is pulled out, the male thread 94 and the female thread (not shown) are threadedly engaged together. The connection between the discharge section 91 and the second valve section 6C is thereby completed.

If a patient can take a fluid substance orally, the fluid substance may be directly administered through the mouth from the bag 2C. In this case, the cap member 96 may be threadedly connected to the discharge body portion 95 such that the distal end portion 97 is located outside.

The other operations are substantially the same as those of Embodiments 1 to 3.

Although a syringe in Embodiment 4 has the same configuration as that of Embodiment 2 described above, the same syringe as the syringe 7A of Embodiment 1 described above may also be employed.

The second valve section 6A and the bag 2A described in Embodiments 1 and 2 may also be employed instead of the fitted portions 18B and 45B, and the fitting portions 24B and 40B.

### [Embodiment 5]

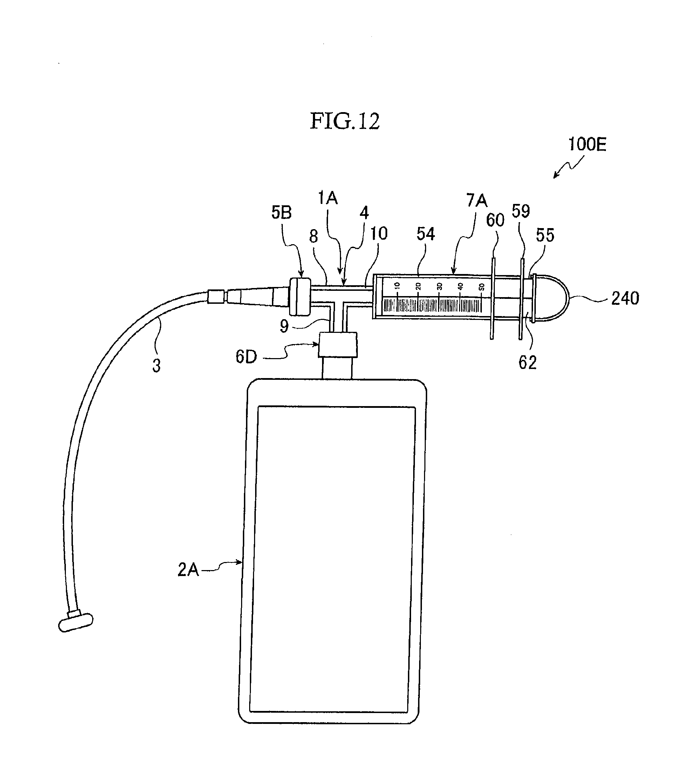

Figures 12 to 16 show a fluid substance transfer device 100E according to Embodiment 5 of the present invention. In Figures 12 to 16, constituent elements assigned with the same reference numerals as those in Figures 1 and 2 indicate the same elements as those in Figures 1 and 2.

As shown in Figure 12, a first valve section 5B is arranged at a position opposing the syringe 7A, and a second valve section 6D is arranged in a direction perpendicular to a line connecting the syringe 7A and the first valve section 5B in a three-way stopcock unit 1E according to Embodiment 5.

Therefore, while fluid resistance increases when a fluid substance is sucked out into the syringe 7A (that is, when a user pulls the plunger rod 62) as compared to Embodiment 1 described above, the fluid resistance can be reduced when the fluid substance is pushed out from the syringe 7A (that is, when a user pushes the plunger rod 62). The positional relationship among the syringe 7A, the first valve section 5B, and the second valve section 6D may be determined as appropriate according to the intended use of the fluid substance transfer device, the type of the fluid substance, or the like.

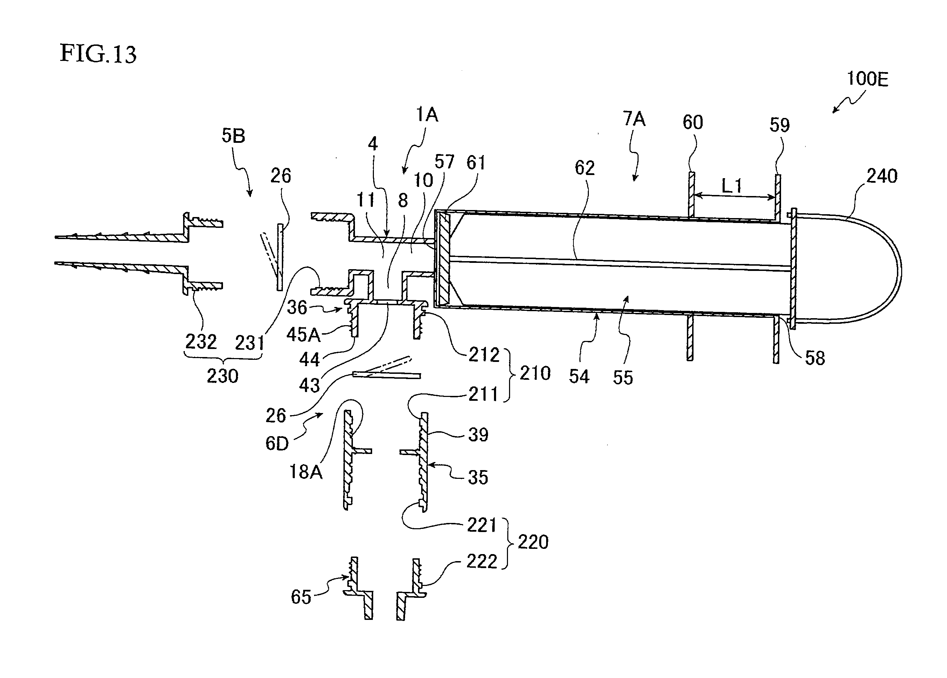

As shown in Figure 13, the three-way stopcock unit 100E according to Embodiment 5 also includes disengagement prevention mechanisms 210, 220, and 230. In the following, the disengagement prevention mechanisms according to Embodiment 5 will be described by using the disengagement prevention mechanism 210 as an example.

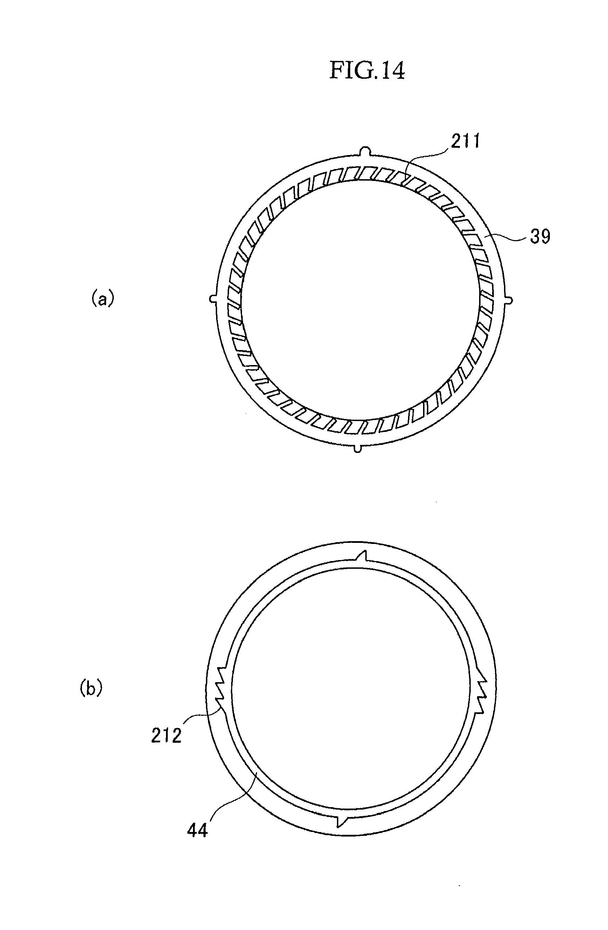

As shown in Figures 13 and 14, the disengagement prevention mechanism 210 is provided in the second valve section 6D. The disengagement prevention mechanism 210 includes a locking claw portion 211 and a locking convex portion 212.

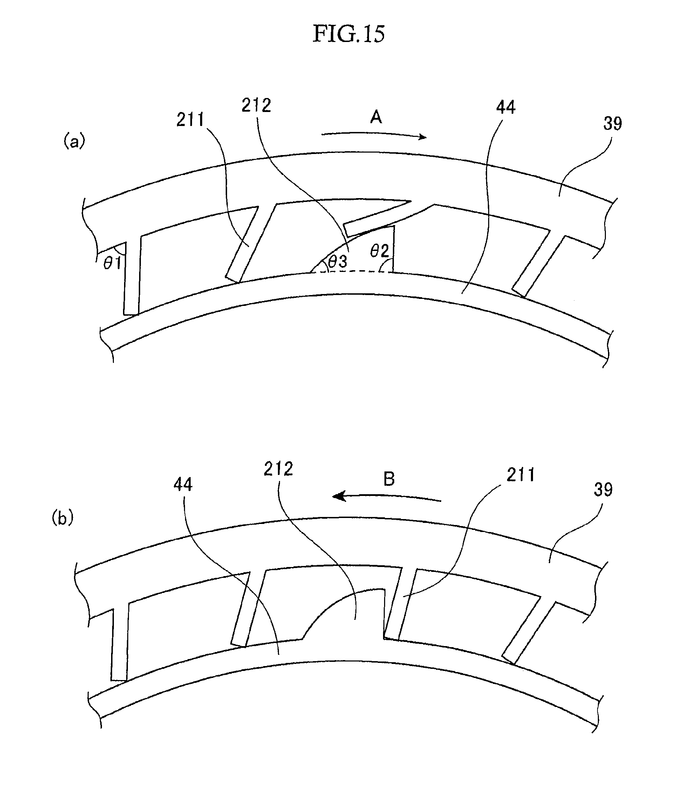

The locking claw portion 211 is formed at a plurality of positions on an inner side surface of the first cylindrical portion 39. Although any number of the locking claw portions 211 may be arranged at any positions, the locking claw portions 211 are preferably formed across an entire circumference of the inner side surface in view of operability. The locking claw portions 211 are formed in an inclined state to a trailing side in the predetermined rotation direction. That is, an inclined angle θ1 (see Figure 15(a)) of the locking claw portion 211 is set to be smaller than 90°.

The locking convex portion 212 is formed at a plurality of positions on an outer side surface of the second cylindrical portion 44. Any number of locking convex portions 212 may be arranged at any positions. In Embodiment 5, the locking convex portions 212 are formed in a substantially triangular shape. In the locking convex portions 212, an angle θ2 on a leading side in a predetermined rotation direction (here, a direction in which the first cylindrical portion 39 is rotated to effect threaded engagement between the first cylindrical portion 39 and the second cylindrical portion 44) is formed to be substantially 90°, and an angle θ3 on the trailing side is formed as an acute angle (see Figure 15(a)).

The principle of the disengagement prevention mechanism 210 will be described based on Figures 13 and 15.

In a similar manner to Embodiment 1 described above, when the second valve section 6D is assembled together, a user fits a distal end portion of the first cylindrical portion 39 with a distal end portion of the second cylindrical portion 44, and rotates the first cylindrical portion 39 in the predetermined direction (the direction of an arrow A in Figure 15(a)). The male thread 45A and the female thread 18A are thereby threadedly engaged together, to cause the distal end portion of the first cylindrical portion 39 to approach a proximal end portion of the second cylindrical portion 44, so that the locking claw portion 211 overlaps with the locking convex portion 212.

At this time, the locking convex portion 212 radially presses the locking claw portion 211. Thus, the locking claw portion 211 is deformed toward the first cylindrical portion 39, thereby allowing the rotation of the first cylindrical portion 39.

Meanwhile, to disengage the first cylindrical portion 39 from the second cylindrical portion 44, the user needs to rotate the first cylindrical portion 39 in the direction of B (see Figure 15(b)). At this time, the locking convex portion 212 cannot radially deform the locking claw portion 211. That is, the locking convex portion 212 is engaged with the locking claw portion 211 to restrict the rotation of the first cylindrical portion 39. As described above, the first cylindrical portion 39 and the second cylindrical portion 44 are threadedly engaged together through the male thread 45A and the female thread 18A. Thus, unless the first cylindrical portion 39 can be rotated, the first cylindrical portion 39 cannot be disengaged from the second cylindrical portion 44. Accordingly, the first and second housings 35 and 36 of the second valve section 6D cannot be separated from each other once the first and second housings 35 and 36 are joined together.

A locking claw portion 221 and a locking convex portion 222 constituting the disengagement prevention mechanism 220, and a locking claw portion 231 and a locking convex portion 232 constituting the disengagement prevention mechanism 230 have the same configuration as those of the disengagement prevention mechanism 210.

In Embodiment 5, the locking claw portion 211 and the locking convex portion 212 are used to constitute the disengagement prevention mechanism 210 as described above, so that the disengagement of the coupling section 13 or the discharge section 65 can be reliably prevented with the simple and inexpensive structure.

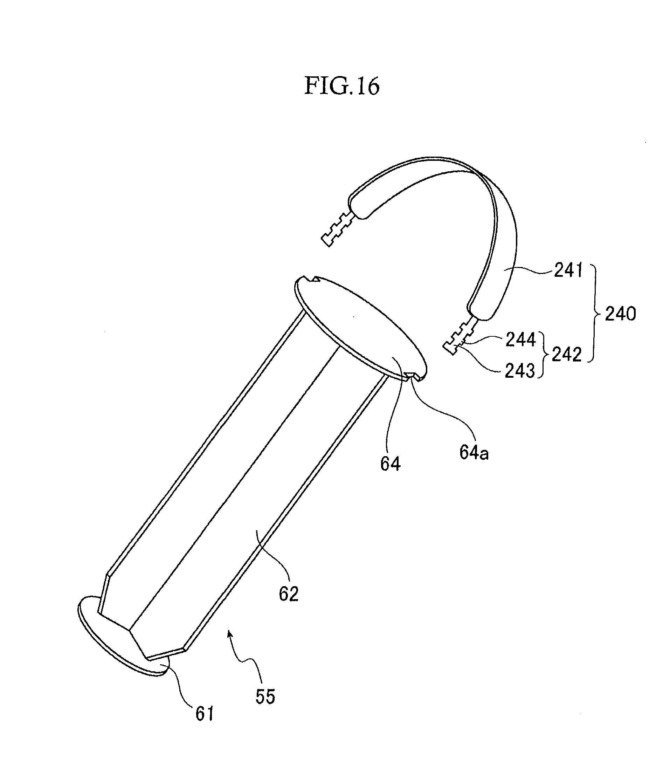

Figure 16 shows the structure of a finger hooking belt member 240 according to Embodiment 5.

As shown in Figure 16, the finger hooking belt member 240 according to Embodiment 5 includes a body portion 241 and a hook portion 242. The body portion 241 and the hook portion 242 are formed integrally with each other in a belt-like shape by using a flexible material. For example, an elastomer resin may be used as the material forming the finger hooking belt member 240.

A length, a width, a hardness or the like of the body portion 241 are determined such that a user easily operates the plunger rod, or does not feel pain in the inserted finger.

The hook portion 242 is provided at both end portions of the body portion 241. The hook portions 242 include narrow recessed portions 243. The hook portions 242 also respectively include a plurality of paired stopper portions 244 and 244 that project in a symmetrical shape. The recessed portions 243 and the stopper portions 244 are alternately formed in the hook portions 242.

The recessed portions 243 are fitted into grooves 64a formed in the proximal end portion 64 of the plunger rod 62. When one of the recessed portions 243 is fitted into the groove 64a, the paired stopper portions 244 and 244 located immediately below the recessed portion 243 are used as the stopper for the finger hooking belt member 240. Since the plurality of recessed portions 243 and the plurality of stopper portions 244 are alternately formed, the diameter of the finger hooking belt member 240 can be freely adjusted.

As described above, in Embodiment 5, the plunger rod 62 and the finger hooking belt member 240 are separately formed, and are joined together by the hook portions 242. Accordingly, while the plunger rod 62 can be formed of a material having enough rigidity, the finger hooking belt member 240 can be formed of a soft material. Good operability can be thereby achieved by preventing a user from feeling pain in the finger or the like.

Furthermore, since the diameter of the finger hooking belt member 240 can be freely adjusted according to the size of the user's finger or the like, the operability can be improved in this point as well.

The structure of the three-way stopcock unit 100E shown in Figure 12 may be applied to the fluid substance transfer device of Embodiments 1 to 4 described above.

Also, the structure of the disengagement prevention mechanism 210 shown in Figures 13 and 14 may be applied to the fluid substance transfer device of Embodiments 1 to 4 described above.

Moreover, the structure of the finger hooking belt member 240 as shown in Figure 16 may be applied to the fluid substance transfer device of Embodiments 1 to 4 described above.

Although the embodiments of the present invention have been described above, the present invention is not limited to Embodiments 1 to 5 described above.

For example, while the check valve of the present invention is used in both the first check valve section 12A and the second check valve section 33A in Embodiments 1 to 5 described above, the check valve of the present invention may be used in only one of the two check valve sections 12A and 33A.

Also, although the anti-slips 32 are provided on the outer circumferential portion of the coupling section 13 in Embodiments 1 to 5 described above, the anti-slips 32 may not be provided in the coupling section 13.

Although the first housing 14 of the first valve section 5 and the second housing 36 of the second valve section 6 are formed integrally with the branch flow channel 4 in Embodiments 1 to 5 described above, the first housing 14, the second housing 36, and the branch flow channel 4 may not be integrally formed, but joined together after being separately formed. Moreover, the syringe 7A and the branch flow channel 4 may be separately formed, and then joined together.

The finger hooking section is not limited to the ring member 63 or the finger hooking belt member 240 describe above, and any finger hooking section may be employed as long as the plunger section 55 can be operated by inserting a user's finger therein.

### Reference Signs List

100A, 100B, 100C, 100D, 100E: Fluid substance transfer device
1A, 1B, 1C, 1D: Three-way stopcock unit
2A, 2B, 2C: Bag
3: Transfer tube
4: Branch flow channel
5A, 5B: First valve section
6A, 6B, 6C, 6D: Second valve section
7A, 7B: Syringe
8: First flow channel
9: Second flow channel
10: Third flow channel
11: Junction
12A: First check valve section (check valve)
14, 35: First housing
15, 37: Valve seat
16, 38: Flow inlet (flow port)
17: First cylindrical portion
19: Opening
20, 36: Second housing
23: Second cylindrical portion
26: Valve element
29: Switching valve
33A: Second check valve section (check valve)
34: Connection section
41, 50, 67, 84: Opening
51: Engagement claw portion
54: Cylinder section
55: Plunger section
61: Plunger head
62: Plunger rod
63: Ring member (insertion hole portion)
64: Proximal end portion
240: Finger hooking belt member
241: Body portion
242: Hook portion

## Claims

1. A three-way stopcock unit comprising:
a branch flow channel that is provided with a first flow channel, a second flow channel, and a third flow channel in which a fluid substance flows, the first flow channel, the second flow channel, and the third flow channel being joined together at one junction;
a syringe that is provided at the third flow channel to supply a suction force for causing the fluid substance to flow into the first flow channel from outside, and an extrusion force for causing the fluid substance to flow outside of the second flow channel, to the branch flow channel;
a first valve section that is provided at an opening end of the first flow channel to prevent the fluid substance from flowing out of the first flow channel; and
a second valve section that is provided at an opening end of the second flow channel to prevent the fluid substance from flowing from the second flow channel;
wherein at least one of the first valve section and the second valve section includes:
a first housing having a first cylindrical portion, a valve seat that closes one of opening ends of the first cylindrical portion, and an opening that is provided in the valve seat;
a second housing having a second cylindrical portion that is inserted into the first cylindrical portion of the first housing from another end of the opening ends; and
a valve element that is formed of an elastic piece so as to be held between the valve seat of the first housing and an opening end portion of the cylindrical portion of the second housing; and
wherein the valve element has a substantially U-shaped cut line formed outside of an outer rim portion of the opening provided in the valve seat of the first housing, and inside of an inner rim portion of the cylindrical portion of the second housing; and
a portion inside of the cut line of the valve element is used as a switching valve, and the valve seat of the first housing and the cylindrical portion of the second housing are closed in a liquid-tight state at a portion outside of the cut line.

2. The three-way stopcock unit according to claim 1, wherein a cylinder section of the syringe and the third flow channel are formed integrally with each other.

3. The three-way stopcock unit according to claim 1 or 2, wherein the first housing provided in the first valve section is formed integrally with the first flow channel.

4. The three-way stopcock unit according to any one of claims 1 to 3, wherein the second housing provided in the second valve section is formed integrally with the second flow channel.

5. The three-way stopcock unit according to any one of claims 1 to 4, wherein at least one of the first valve section and the second valve section includes,
a cylindrical connection member that is provided so as to be threadedly engaged or fitted with a connection section of the other element, and
an engagement claw portion that is provided in the connection member to prevent disengagement of the other element by engagement with the other element when the connection member is connected to the other element.

6. The three-way stopcock unit according to claim 5, wherein;
the connection member is formed as a female member into which the other element can be inserted, and
the engagement claw portion is provided projecting on an inner side surface of the connection member so as to form an acute angle in a direction in which the other element is inserted.

7. The three-way stopcock unit according to any one of claims 1 to 4;
wherein at least one of the first valve section and the second valve section further comprising;
a configuration in which the first cylindrical portion is inserted into and threadedly engaged with the second cylindrical portion while the first cylindrical portion is being rotated in a predetermined direction by using a male thread formed on one of an inner side surface of the first cylindrical portion and an outer side surface of the second cylindrical portion, and a female thread formed in another surface of the inner side surface and the outer side surface; and
a disengagement prevention mechanism that allows the rotation of the first cylindrical portion only in the predetermined direction, and thereby prevents disengagement of the second cylindrical portion from the first cylindrical portion.

8. The three-way stopcock unit according to claim 7, wherein;
the disengagement prevention mechanism has one or a plurality of locking claw portions formed on one of the inner side surface of the first cylindrical portion and the outer side surface of the second cylindrical portion, and one or a plurality of locking convex portions formed on another surface of the inner side surface and the outer side surface,
the locking claw portion being formed in an inclined state to a trailing side in the predetermined direction, and
the locking convex portion allowing the rotation of the first cylindrical portion by pressing and deforming the locking claw portion in a direction substantially perpendicular to the rotation direction when the first cylindrical portion rotates in the predetermined direction, and restricting the rotation of the first cylindrical portion by engagement with the locking claw portion when the first cylindrical portion rotates in a direction opposite to the predetermined direction.

9. The three-way stopcock unit according to any one of claims 1 to 8, wherein the plunger section of the syringe includes:
a substantially disk-shaped resin plunger head that slides within the cylinder section in a liquid-tight state; and
a resin plunger rod that is formed integrally with the plunger head.

10. The three-way stopcock unit according to any one of claims 1 to 9, wherein the plunger section of the syringe includes:
a plunger head that slides within the cylinder section;
a plunger rod that is connected to the plunger head;
a finger hooking section that is provided on a proximal end portion of the plunger rod to effect an axial movement operation of the plunger rod by a user's finger inserted therein; and
two flange portions that are axially provided parallel to each other on the cylinder section of the syringe so as to have a distance therebetween into which a user's finger is inserted.

11. The three-way stopcock unit according to claim 10, wherein the finger hooking section is a ring member that is provided projecting on the plunger rod.

12. The three-way stopcock unit according to claim 10, wherein:
the finger hooking section includes a belt-like body portion that is formed of a flexible material, and a hook portion that is provided at both ends of the body portion to maintain the finger hooking section by fitting into a groove formed in the proximal end portion of the plunger rod;
the hook portion having the one or the plurality of narrow recessed portions, and one or a plurality of wide stopper portions that come into abutment against the proximal end portion from a lower side within the groove when the recessed portion is fitted into the groove; and
the recessed portion and the stopper portion being alternately provided.

13. A check valve comprising:
a first housing having a first cylindrical portion, a valve seat that closes one of opening ends of the first cylindrical portion, and an opening that is provided in the valve seat;
a second housing having a second cylindrical portion that is inserted into the first cylindrical portion of the first housing from another end of the opening ends; and
a valve element that is formed of an elastic piece so as to be held between the valve seat of the first housing and an opening end portion of the cylindrical portion of the second housing;
wherein the valve element has a substantially U-shaped cut line formed outside of an outer rim portion of the opening provided in the valve seat of the first housing, and inside of an inner rim portion of the cylindrical portion of the second housing; and
a portion inside of the cut line of the valve element is used as a switching valve, and the valve seat of the first housing and the cylindrical portion of the second housing are closed in a liquid-tight state at a portion outside of the cut line.

14. A fluid substance transfer device comprising:
the three-way stopcock unit according to any one of claims 1 to 12;
a storage vessel that stores a fluid substance flowing into the three-way stopcock unit from the first flow channel, and is connected to the second valve section; and
a tube that is connected to the first valve section to supply the fluid substance into a human body.
